# EUROPÄISCHE PATENTSCHRIFT

(11) **EP 0 639 556 B1**
(45) Veröffentlichungstag und Bekanntmachung des Hinweises auf die Patenterteilung: **05.03.1997**
(21) Anmeldenummer: 94112737.5
(22) Anmeldetag: 16.08.1994
(51) Int. Cl.: C07C 209/74, C07B 39/00

(54) **Verfahren zur Herstellung von Trifluormethylanilinen**
Process for the preparation of trifluoromethylanilines
Procédé pour la préparation de trifluorométhylanilines

(30) Priorität: 20.08.1993 DE 4328028
(43) Veröffentlichungstag der Anmeldung: 22.02.1995
(73) Patentinhaber: HOECHST AKTIENGESELLSCHAFT, 65929 Frankfurt am Main (DE)
(72) Erfinder: Appel, Wolfgang, Dr., D-65779 Kelkheim/Ts. (DE); Siegemund, Günter, Dr., D-65719 Hofheim/Ts (DE)

(56) Entgegenhaltungen:
- EP-A- 0 152 310
- GB-A- 955 898
- JOURNAL OF FLUORINE CHEMISTRY., Bd. 52, Nr. 2, 15.April 1991, LAUSANNE CH, Seiten 107-116, HENRY C. LIN ET AL.: "Rearrangement of N-(trifluoromethyl)anthraniloyl fluoride to 2-(trifluoromethyl)aniline - kinetic and mechanistic observations"
- A.Michel 'CHIMIE MINERALE', 1964, S.216-217
- DICTIONNAIRE LE PETIT ROBERT, 1979, S.1494
- LE GRAND DICTIONNAIRE LANGENSCHEIDT, FR-DE, 1979, S.744

## Beschreibung

Die vorliegende Erfindung betrifft ein Verfahren zur Herstellung von 4-Trifluormethylanilinen.

Trifluormethylaniline und insbesondere 4-Trifluormethylaniline sind wertvolle Zwischenprodukte für den Aufbau von Wirkstoffen im Pharma- und Pflanzenschutzbereich.

Die Herstellung von p-Trifluormethylanilin ausgehend von 4-Chlortrifluormethylbenzol durch Aminolyse in Gegenwart von Kupfer(I)chlorid und Kaliumfluorid ist beschrieben (L. P. Seiwell, J. Org. Chem. 44, 4731 bis 4733 (1979)). Jedoch sind die Umsätze niedrig und die Ausbeuten entsprechend zu gering für ein technisches Verfahren.

Ebenso sind einige Verfahren beschrieben, p-Trifluormethylanilin durch Reduktion der entsprechenden Nitrovorstufe herzustellen (z. B. J. Org. Chem. 26, 1477 bis 1480 (1961), J. Amer. Chem. Soc. 69, 2346 bis 2350 (1947)). Aber die Zugänglichkeit von 4-Nitrotrifluormethylbenzol ist sehr schlecht, da die Nitrierung von Benzoltrifluorid praktisch ausschließlich (> 96 %) die m-Verbindung liefert.

Beschrieben ist auch eine Methode, um durch radikalische Addition von Trifluormethylbromid an elektronenreiche Aromaten die entsprechenden Trifluormethylarylderivate zu erhalten (J. Chem. Soc., Perkin 1, 2293 bis 2299 (1990)). Die Reaktion ist jedoch wenig stellungsselektiv und liefert auch nur mäßige Ausbeuten. Weitere in der neueren Literatur beschriebene Methoden kommen ebenfalls aufgrund sehr teurer Reagenzien (Tetrahedron Lett. 31, 3579 bis 3582 (1990)) oder aufwendiger Reaktionsschritte (J. Org. Chem. 54, 2873 bis 2877 (1989)) für eine industrielle Anwendung nicht in Frage.

Auch ausgehend vom p-Trichlormethylphenylisocyanat sind zwei Verfahren beschrieben. Bei zwei älteren Patentschriften wird zunächst aus Trichlormethylphenylisocyanat das Trifluormethylphenylcarbamoylfluorid hergestellt, woraus nach Entfernen der Flußsäure in Gegenwart von Xylol das entsprechende Isocyanat thermisch zurückgewonnen wird (GB-A-955 898). Anschließend wird in aufwendiger Weise (FR-A-1 545 142) das Trifluormethylphenylisocyanat in ein organisches Lösungsmittel überführt und schließlich mit konz. (98 %) Schwefelsäure die Isocyanatgruppierung hydrolysiert.

Bei der neueren Patentschrift (EP-A-0 152 310) wurde das Verfahren dahingehend vereinfacht, daß man Chlor-Fluor-Austausch und Hydrolyse in einem Schritt durchführt, indem man der Flußsäure vorzugsweise eine bestimmte (mindestens molare) Menge Wasser zusetzt und die Aufarbeitung des Reaktionsgemisches in definierter Weise vornimmt.

Andererseits ist aus der neueren Literatur (Lin, Cotter, Bieron, Krishnamurti, J. Fluorine Chem. 52, 107 bis 116 (1991)) bekannt, daß sich aus 2-Trichlormethylphenylisocyanat in wasserfreier Flußsäure in einer bis zu diesem Zeitpunkt unbekannten Solvolysereaktion unter Entstehen von Carbonylfluorid direkt das Hydrofluorid des 2-Trifluormethylanilins bildet. Die Anwendung der gleichen Reaktionsbedingungen auf Phenylisocyanat und p-Trifluormethylphenylisocyanat führte jedoch in hohem Maß zur Bildung der entsprechenden Diarylharnstoffe was im Fall des ortho-substituierten Derivate aus sterischen Gründen verhindert wird. Aus der US-A-4 481 370 ist bekannt, daß die Umsetzung verschiedener Phenylcarbaminsäurefluoride zu den Arylaminhydrofluoriden bei Raumtemperatur oder erhöhter Temperatur möglich ist, wenn man zuvor bei niedrigen Temperaturen (um 0°C) aus den Phenylisocyanaten die Phenylcarbaminsäurefluoride erzeugt. Allerdings wird dort beschrieben, daß zur Erzielung akzeptabler Reaktionszeiten der Zusatz von Wasser benötigt wird. Hierzu kommt, daß die berichteten Ausbeuten - außer beim 2-Trifluor-methylanilin - zu gering sind, um das Verfahren ökologisch und ökonomisch attraktiv erscheinen zu lassen.

Wegen der allgemeinen Bedeutung und der vielseitigen Verwendbarkeit dieser Stoffklasse stellt es eine lohnende Aufgabe dar, ein neues Verfahren zur Synthese dieser Verbindungen bereitzustellen, das die beschriebenen Nachteile vermeidet, und technisch einfach durchzuführen ist.

Die Aufgabe wird gelöst durch ein Verfahren zur Herstellung Von Trifluormethylanilinen der Formel (I), 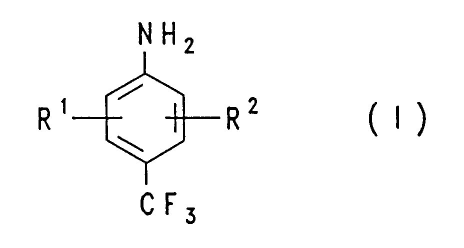 worin
R¹ und R² unabhängig voneinander Wasserstoff, Halogen, (C₁-C₄)-Alkyl, Hydroxy, Alkoxy, Alkylthio, Carboxy, Nitro oder Cyanogruppe bedeuten, indem man Verbindungen der Formel (II) 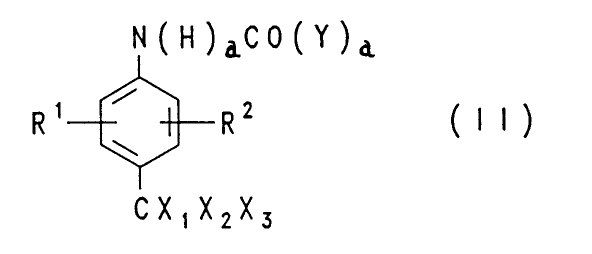
worin
X₁, X₂, X₃ jeweils gleich oder verschieden Halogenatome bedeuten,
a gleich Null oder 1 ist,
Y gleich Fluor, Chlor oder Brom ist und
R¹ und R² die definierte Bedeutung besitzen, mit wasserfreier Flußsäure bei 20 bis 130°C umsetzt, wobei das molare Verhältnis von wasserfreier Flußsäure zu Verbindung der Formel (II) zwischen 5 und 50 liegt, und die entstehenden Anilinhydrofluoride mit einem Alkalimetallhydroxid in das freie Amin überführt, dadurch gekennzeichnet, daß man wasserfreie Flußsäure ohne Zusatz von Wasser verwendet.

Es wurde überraschenderweise gefunden, daß auch an p-Trihalogenmethylpbenylisocyanaten in wasserfreier Flußsäure in einem Schritt in hervorragenden Ausbeuten Halogen-Fluoraustausch und Solvolyse der Isocyanatgruppe zu den 4-Trifluormethylphenylanilin-hydrofluoriden erfolgen. Diese können dann nach Abdestillieren der wasserfreien Flußsäure durch einfache Neutralisation in das freie Amin überführt werden. Der Zusatz von Wasser zur Flußsäure wird vermieden, wodurch diese unproblematisch und mit Ausnahme der für die Reaktion erforderlichen Equivalente nahezu vollständig recyclisiert werden kann.

Als Edukte kommen neben Phenylisocyanaten auch die entsprechenden Phenylcarbamoylhalogenide in Frage, wobei diese in situ zu den Phenylcarbamoylfluoriden umgesetzt werden - sofort nicht direkt das Carbamoylfluorid eingesetzt wird - und dann dem gleichen Solvolyseschritt unterliegen.

Das Verfahren ist ökologisch sehr vorteilhaft, da die Flußsäure praktisch quantitativ recyclisierbar ist.

Das Verfahren ist mit allen Trihalogenmethylderivaten durchführbar, technisch von besonderem Interesse ist jedoch der Einsatz der entsprechenden Trichlorverbindungen.

Sehr interessante Einsatzstoffe sind 4-Trichlormethylphenylisocyanat, 2-Chlor-4-trichlormethylphenylisocyanat, 2,6-Dichlor-4-trichlormethylphenylisocyanat sowie die entsprechenden Carbamoylhalogenide.

Zur erfindungsgemäßen Ausführung der Reaktion muß mindestens die fünffach molare Menge an Flußsäure pro Mol Isocyanat bzw. Carbamoylhalogenid verwendet werden, jedoch setzt man vorzugsweise 10 bis 40 Equivalente ein, was infolge der leichten Recyclisierbarkeit von wasserfreier Flußsäure unproblematisch ist.

Die Reaktion läßt man im allgemeinen bei Temperaturen zwischen 20 und 130°C ablaufen. In vielen Fällen hat es sich bewährt bei 20 bis 80°C, insbesondere 35 und 70°C zu arbeiten. Die Reaktionsdauer hängt vom Substrat und von der Reaktionstemperatur ab und liegt zwischen 0,5 bis 5 Stunden.

Als Reaktionsgefäße finden Druckbehälter aus Edelstahl oder anderen geeigneten Materialien Verwendung, die mit einem Rückflußkühler und einem dahinter befindlichen Ventil versehen sein sollten, um das Abgasen von Chlorwasserstoff und Carbonylfluorid während der Reaktion zu ermöglichen.

Nach Beendigung der Reaktion werden zunächst Chlorwasserstoff und Carbonylfluorid vollständig abgegast. Anschließend destilliert man bei ca. 30°C die überschüssige wasserfreie Flußsäure, die ohne weitere Reinigung wieder eingesetzt werden kann, möglichst vollständig ab. Nach Öffnen des Autoklaven kann das Reaktionsgemisch als solches entnommen werden oder zunächst durch Zusatz eines geeigneten Lösungsmittels wie Ethylacetat, Methylenchlorid, Methyl-t-butylketon, Toluol o. a. in Lösung überführt werden.

Aus dem in einem der angeführten Lösungsmittel befindlichen Reaktionsgemisch erhält man nach Einstellen des pH-Wertes auf 8 bis 10 das freie Anilin, das dann durch einfache Destillation in hohen Ausbeuten gewonnen werden kann.

Die folgenden Beispiele dienen zur Erläuterung des erfindungsgemäßen Verfahrens, ohne es jedoch darauf zu beschränken.

### Beispiele:

### 1) 4-Trifluormethylanilin

In einem zuvor im Inertgasstrom getrockneten 2 l Edelstahlautoklaven mit Rührer, Rückflußkühler und darüber befindlichem Absperrventil werden 236 g (1 Mol) 4-Trichlormethylphenylisocyanat vorgelegt und anschließend 600 g (30 Mol) wasserfreie Flußsäure (Wassergehalt < 0,1 %) zugegeben.

Man erhitzt jetzt für 4 Stunden auf 70°C. Während der Reaktionszeit öffnet man ab und zu das Absperrventil und erniedrigt den Innendruck jeweils auf ca. 10 bar durch Abgasen von Chlorwasserstoff und Carbonylfluorid.

Am Ende der Reaktionszeit läßt man auf 30 bis 35°C Innentemperatur abkühlen und gast bei dieser Temperatur die Flußsäure möglichst vollständig ab. Nach Öffnen des Reaktors löst man das Reaktionsgemisch in 500 ml Ethylacetat und transferiert es in ein Rührgefäß, in dem 500 ml einer Eis/Wasser Mischung vorgelegt sind. Unter kräftigem Rühren stellt man durch Zusatz einer 30 %igen wäßrigen Kaliumhydroxidlösung den pH-Wert der Mischung auf 8 bis 10 ein. Nach Abtrennen der organischen Oberphase wäscht man diese noch je einmal mit Wasser und Kochsalzlösung und trocknet über Natriumsulfat. Durch fraktionierte Destillation im Vakuum erhält man 135 g (83,8 % der Theorie) 4-Trifluormethylanilin (Siedepunkt 86°C/14 mm Hg) dessen Reinheit (GC > 99,8 %) und Identität geprüft wurden.
(¹H-NMR (CDCl₃): δ = 7,3, 2 H, d = 8,7 Hz; δ = 6,6, 2 H, d = 8,7 Hz; δ = 3,9 br s, 2 H)

### 2) 2-Chlor-4-trifluormethylaninlin

In gleicher Weise wie bei Beispiel 1 werden 135 g (0,5 Mol) 2-Chlor-4-trichlormethylphenylisocyanat und 500 g wasserfreie HF in einem Druckgefäß 4 Stunden bei 70°C zur Reaktion gebracht und anschließend völlig analog aufgearbeitet. Man erhält das Produkt (76 g, 78 % d. Th.) wiederum durch fraktionierte Destillation. Die Identität und Reinheit wurden durch GC-NMR
(¹H- NMR (CDCl₃): δ = 7,5, 1 H, br s; δ = 7,2, 1 H, br s; δ = 6,6, 1 H, d, 8,6 Hz; δ = 4,35, 2 H, br s) bewiesen.

### 3) 2,6-Dichlor-4-trifluormethylanilin

In gleicher Weise wie bei Beispiel 1 werden 151,1 g (0,5 Mol) 2,6-Dichlor-4-trichlormethylanilin 5 Stunden bei 70°C mit 500 g wasserfreier Flußsäure behandelt. Nach analoger Aufarbeitung erhält man durch fraktionierte Destillation das erwünschte Produkt in guter Ausbeute (81 g, 70 % d. Th.). Die Identität wurde durch Spektren und Schmelzpunkt (35 bis 36°C) überprüft.

## Patentansprüche

1. Verfahren zur Herstellung von Trifluormethylanilinen der Formel (I), 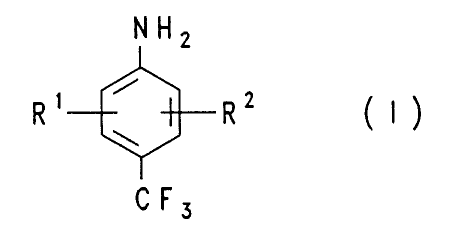 worin
R¹ und R² unabhängig voneinander Wasserstoff, Halogen, (C₁-C₄)-Alkyl, Hydroxy, Alkoxy, Alkylthio, Carboxy, Nitro oder Cyanogruppe bedeuten, indem man Verbindungen der Formel (II) 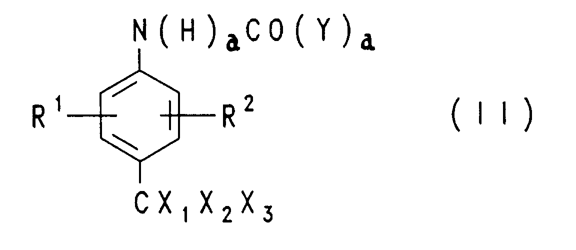
worin
X₁, X₂, X₃ jeweils gleich oder verschieden Halogenatome bedeuten,
a gleich Null oder 1 ist,
Y gleich Fluor, Chlor oder Brom ist und
R¹ und R² die definierte Bedeutung besitzen, mit wasserfreier Flußsäure bei 20 bis 130°C umsetzt, wobei das molare Verhältnis von wasserfreier Flußsäure zu Verbindung der Formel (II) zwischen 5 und 50 liegt, und die entstehenden Anilinhydrofluoride mit einem Alkalimetallhydroxid in das freie Amin überführt, dadurch gekennzeichnet, daß man wasserfreie Flußsäure ohne Zusatz von Wasser verwendet.

2. Verfahren nach Anspruch 1, dadurch gekennzeichnet, daß in Formel (II) a gleich Null ist.

3. Verfahren nach Anspruch 1 oder 2, dadurch gekennzeichnet, daß in Formel (II) X₁, X₂ und X₃ Chloratome sind.

4. Verfahren nach mindestens einem der Ansprüche 1 bis 3, dadurch gekennzeichnet, daß in Formel (II) R¹ und R² Wasserstoff oder Chlor bedeutet.

5. Verfahren nach mindestens einem der Ansprüche 1 bis 4, dadurch gekennzeichnet, daß Formel (II) 4-Trichlormethylphenylisocyanat darstellt.

6. Verfahren nach mindestens einem der Ansprüche 1 bis 5, dadurch gekennzeichnet, daß das molare Verhältnis von wasserfreier Flußsäure zu Verbindung der Formel (II) zwischen 10 und 40 liegt.

7. Verfahren nach mindestens einem der Ansprüche 1 bis 6, dadurch gekennzeichnet, daß die Umsetzung mit wasserfreier Flußsäure bei einer Temperatur von 20 bis 80°C, bevorzugt 35 und 70°C durchgeführt wird.

8. Verfahren nach mindestens einem der Ansprüche 1 bis 7, dadurch gekennzeichnet, daß als Base Kaliumhydroxid verwendet wird.

9. Verfahren nach mindestens einem der Ansprüche 1 bis 8, dadurch gekennzeichnet, daß die wasserfreie Flußsäure recycliert wird.

## Claims

1. A process for preparing trifluoromethylanilines of the formula (I), 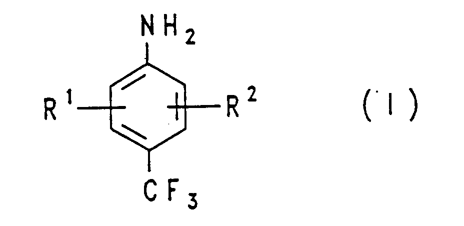 in which
R¹ and R², independently of each other, are hydrogen, halogen, (C₁-C₄)-alkyl, hydroxyl, alkoxy, alkylthio, carboxyl, or a nitro or cyano group, by reacting compounds of the formula (II) 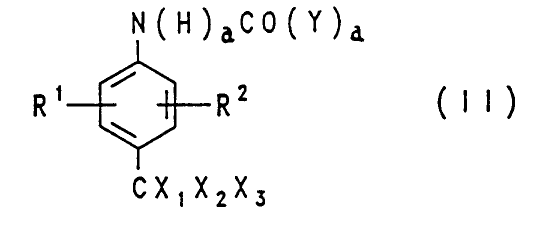
in which
X₁, X₂ and X₃ are in each case, identically or differently, halogen atoms,
a is zero or 1,
Y is fluorine, chlorine or bromine, and
R¹ and R² have the defined meaning, with anhydrous hydrofluoric acid at from 20 to 130°C, the molar ratio of anhydrous hydrofluoric acid to compound of the formula (II) being between 5 and 50, and converting the resulting aniline hydrofluorides with an alkali metal hydroxide into the free amine, which comprises using anhydrous hydrofluoric acid without addition of water.

2. The process as claimed in claim 1, wherein a is zero in formula (II).

3. The process as claimed in claim 1 or 2, wherein X₁, X₂ and X₃ are chlorine atoms in formula (II).

4. The process as claimed in at least one of claims 1 to 3, wherein R¹ and R² are hydrogen or chlorine in formula (II).

5. The process as claimed in at least one of claims 1 to 4, wherein formula (II) represents 4-trichloromethylphenyl isocyanate.

6. The process as claimed in at least one of claims 1 to 5, wherein the molar ratio of anhydrous hydrofluoric acid to compound of the formula (II) is between 10 and 40.

7. The process as claimed in at least one of claims 1 to 6, wherein the reaction with anhydrous hydrofluoric acid is carried out at a temperature of from 20 to 80°C, preferably between 35 and 70°C.

8. The process as claimed in at least one of claims 1 to 7, wherein potassium hydroxide is used as the base.

9. The process as claimed in at least one of claims 1 to 8, wherein the anhydrous hydrofluoric acid is recycled.

## Revendications

1. Procédé de préparation de trifluorométhylanilines de formule (I), 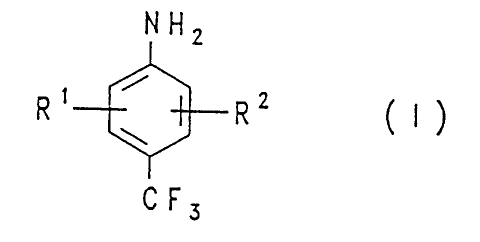 dans laquelle
R¹ et R², indépendamment l'un de l'autre, représentent l'hydrogène, halogène, alkyle en C₁-C₄, hydroxy, alcoxy, alkylthio, carboxy, nitro ou cyano, selon lequel on fait réagir des composés de formule (II) 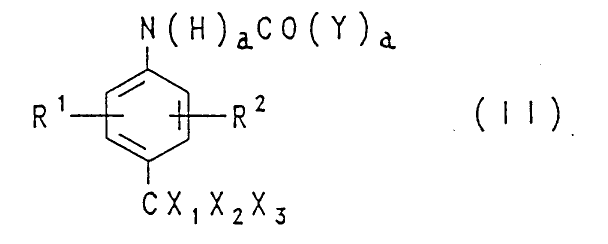
dans laquelle
X₁, X₂, X₃ représentent chacun des atomes d'halogènes identiques ou différents,
a est 0 ou 1,
Y est fluor, chlore ou brome et
R¹ et R² possèdent la signification définie, avec du fluorure d'hydrogène anhydre à 20 jusqu'à 130°C, la proportion molaire du fluorure d'hydrogène anhydre au composé de formule (II) étant comprise entre 5 et 50, et on transforme les fluorhydrates d'anilines résultants au moyen d'hydroxyde de métal alcalin en l'amine libre, caractérisé en ce qu'on utilise du fluorure d'hydrogène anhydre sans addition d'eau.

2. Procédé selon la revendication 1, caractérisé en ce que dans la formule (II), a vaut 0.

3. Procédé selon la revendication 1 ou 2, caractérisé en ce que dans la formule (II) X₁, X₂ et X₃ sont des atomes de chlore.

4. Procédé selon au moins une des revendications 1 à 3, caractérisé en ce que dans la formule (II) R¹ et R² représentent l'hydrogène ou le chlore.

5. Procédé selon au moins une des revendications 1 à 4, caractérisé en ce que la formule (II) représente l'isocyanate de 4-trichlorométhylphényle.

6. Procédé selon au moins une des revendications 1 à 5, caractérisé en ce que la proportion molaire du fluorure d'hydrogène anhydre au composé de formule (II) est comprise entre 10 et 40.

7. Procédé selon au moins une des revendications 1 à 6, caractérisé en ce que la réaction avec le fluorure d'hydrogène anhydre est effectuée à une température de 20 à 80°C, de préférence entre 35 et 70°C.

8. Procédé selon au moins une des revendications 1 à 7, caractérisé en ce que de l'hydroxyde de potassium est utilisé comme base.

9. Procédé selon au moins une des revendications 1 à 8, caractérisé en ce que le fluorure d'hydrogène anhydre est recyclé.
